# EUROPÄISCHE PATENTANMELDUNG

(11) **EP 3 944 262 A1**
(43) Veröffentlichungstag der Anmeldung: **26.01.2022**
(21) Anmeldenummer: 21187206.4
(22) Anmeldetag: 22.07.2021
(51) Int. Cl.: H01B 9/04, H01B 7/16, H01B 13/32, H01B 13/004, H02G 5/06

(54) **VERFAHREN ZUM HERSTELLEN EINER ZWEIPOLIG ELEKTRISCH LEITENDEN ZUGSTANGE SOWIE ELEKTRISCH LEITENDE ZUGSTANGE UND MEDIZINISCHES INSTRUMENT**

(30) Priorität: 22.07.2020 DE 102020119298
(71) Anmelder: Karl Storz SE & Co. KG, 78532 Tuttlingen (DE)
(72) Erfinder: BLOCHER, Martin, 78532 Tuttlingen (DE); GRÜNER, Sven Axel, 78532 Tuttlingen (DE); HOLZER, Judith, 78532 Tuttlingen (DE); KÄRCHER, Daniel, 78532 Tuttlingen (DE); LÄNGLE, Dominik, 78532 Tuttlingen (DE); MERZ, Robin, 78532 Tuttlingen (DE); SCHNEIDER, Janosz, 78532 Tuttlingen (DE); SCHNEIDER, Sven, 78532 Tuttlingen (DE); STEFAN, Jochen, 78532 Tuttlingen (DE); UNGER, Tobias, 78532 Tuttlingen (DE)

(57) **Zusammenfassung**

Die Erfindung betrifft ein Verfahren zum Herstellen einer zweipolig elektrisch leitenden Zugstange mit einer elektrisch leitenden Stange in einem elektrisch leitenden Rohr, wobei ein Rohrinnendurchmesser größer als ein Stangendurchmesser ist, mit den folgenden Schritten:
- Einführen des Rohrs in eine Haltevorrichtung, wobei die Haltevorrichtung derart ausgestaltet ist, dass die Haltevorrichtung das Rohr an einer Rohraußenseite kontaktiert,
- Befestigen der Stange in einer Spannvorrichtung, wobei die Stange mittels der Spannvorrichtung beim Befestigen derart in Bezug zur Haltevorrichtung ausgerichtet wird, dass die befestigte Stange mittels der Spannvorrichtung mittig in das Rohr einführbar ist,
- mittiges Einführen der befestigten Stange in das Rohr, wobei eine Rohrinnenseite und eine Stangenaußenseite der eingeführten Stange vollständig durch einen Zwischenraum getrennt sind, sodass die Stange und das Rohr frei von Kontakt sind, wobei die Stange entweder vor oder nach dem Einführen mittels der Spannvorrichtung gespannt wird, sodass eine Zugkraft längs der Stange wirkt, und
- Ausfüllen des Zwischenraums mit einer erhärtenden Flüssigkeit bzw. einem erhärtenden Granulat, insb. Kunststoffgranulat, wobei die erhärtende Flüssigkeit bzw. das erhärtende Granulat wenigstens im erhärteten Zustand isolierend wirkt, sodass die Stange mittig in dem Rohr fixiert und die zweipolig elektrisch leitende Zugstange erhalten wird

sowie eine elektrisch leitende Zugstange und ein medizinisches Instrument, insbesondere Zangensystem, speziell HF-Zangensystem, oder auch Endoskop oder Exoskop.

## Beschreibung

Die Erfindung betrifft ein Verfahren zum Herstellen einer zweipolig elektrisch leitenden Zugstange mit einer elektrisch leitenden Stange in einem elektrisch leitenden Rohr sowie eine elektrisch leitende Zugstange und ein medizinisches Instrument.

Im Stand der Technik sind in einigen Vorrichtungen Stangen-artige Bauteile bekannt, die einen Teil eines Stromkreises bilden, beispielsweise Zugstangen.

Eine Möglichkeit zur Stromleitung ist das Einbringen einer Aussparung in dem säulenförmigen Bauteil verbunden mit dem Verlegen von Kabeln. Dies schwächt jedoch die mechanische Stabilität des Bauteils und erfordert zahlreiche Arbeitsschritte.

Eine andere Möglichkeit zur Verwendung des Stangen-artige Bauteils selbst als Teil eines elektrischen Stromkreises ist die Verwendung einer zentralen Stange sowie eines umgebenden Rohrs, wobei Stange und Rohr gegeneinander elektrisch isoliert sind. In diesem Fall kann beispielsweise die Stange den Pluspol sowie das Rohr den Minuspol bilden oder umgekehrt.

Als Isolierung zwischen Stange und Rohr kann ein Schrumpfschlauch verwendet werden, welcher sich um die Stange legt und diese somit gegenüber dem Rohr isoliert. Aufgrund der Toleranzen der schwankenden Schrumpfrate von Schrumpfschläuchen kann sich ein Spalt zwischen den beiden elektrischen Polen bilden. Weiterhin ist eine exakte Abmessung des Schrumpfschlauchs gegenüber dem Rohrinnendurchmesser schwierig, sodass es leicht zu Lücken zwischen dem die Stange umgebenden Schrumpfschlauch und dem Rohr kommt. In diese Lücke können Schmutzpartikel eindringen. Dies ist insbesondere in biomedizinischen Anwendungen nachteilig.

Eine weitere Möglichkeit ist das Einführen der Stange in das Rohr sowie das anschließende Verfüllen des sich bildenden Zwischenraums mit einem Kunststoff. Bei sehr präzisen Vorrichtungen in Leichtbauweise ist jedoch der Zwischenraum im Verhältnis zur Länge klein und das Rohr dünnwandig ausgestaltet. Durch das Eigengewicht des Rohrs kann sich dieses geringfügig verbiegen und somit die Stange während des Fertigungsprozesses kontaktieren. Weiterhin weisen Rohre und Stangen fertigungsbedingt immer eine gewissen Biegung auf. Durch eine leichte Biegung von Rohr und/oder Stange entstehen so kontaktierende Bereiche.

Die kontaktierenden Bereiche von Stange und Rohr können nicht mit Kunststoff verfüllt und isoliert werden, wodurch diese im Betrieb eine Leitfähigkeit zwischen Rohr und Stange ermöglichen und so Kurzschlüsse verursachen.

Aufgabe der Erfindung ist es, den Stand der Technik zu verbessern.

Gelöst wird die Aufgabe durch ein Verfahren zum Herstellen einer zweipolig elektrisch leitenden Zugstange mit einer elektrisch leitenden Stange in einem elektrisch leitenden Rohr, wobei ein Rohrinnendurchmesser größer als ein Stangendurchmesser ist, mit den folgenden Schritten:
- Einführen des Rohrs in eine Haltevorrichtung, wobei die Haltevorrichtung derart ausgestaltet ist, dass die Haltevorrichtung das Rohr an einer Rohraußenseite kontaktiert,
- Befestigen der Stange in einer Spannvorrichtung, wobei die Stange mittels der Spannvorrichtung beim Befestigen derart in Bezug zur Haltevorrichtung ausgerichtet wird, dass die befestigte Stange mittels der Spannvorrichtung mittig in das Rohr einführbar ist (ggf. kann sich die Stange beim Einspannen bereits innerhalb des Rohres befinden und dann nur noch ausgerichtet werden),
- mittiges Einführen bzw. Ausrichten der befestigten Stange in das bzw. in dem Rohr, wobei eine Rohrinnenseite und eine Stangenaußenseite der eingeführten Stange vollständig durch einen Zwischenraum getrennt sind, sodass die Stange und das Rohr frei von Kontakt sind, wobei die Stange entweder vor oder nach dem Einführen bzw. Ausrichten mittels der Spannvorrichtung gespannt wird, sodass eine Zugkraft längs der Stange wirkt, und
- Ausfüllen des Zwischenraums mit einer erhärtenden Flüssigkeit, wobei die erhärtende Flüssigkeit wenigstens im erhärteten Zustand isolierend wirkt, sodass die Stange mittig in dem Rohr fixiert und die zweipolig elektrisch leitende Zugstange erhalten wird.

Somit wird insbesondere ein Verlegen von Kabeln unnötig, wodurch ein Fertigungsaufwand reduziert wird. Weiterhin kann so auf die Verwendung eines Schrumpfschlauches verzichtet werden, sodass ein Spalt zwischen den elektrischen Polen vermieden werden kann, in welchen sonst Verschmutzung eindringen kann.

Folgendes Begriffliche sei erläutert:
Eine "zweipolig elektrisch leitende Zugstange" ist insbesondere ein säulen- oder stabförmiger Gegenstand, welcher Zugkräfte übertragen kann sowie zwei elektrisch leitende Pole aufweist.

Unter einer "Stange" wird ein Gegenstand verstanden, welcher im Verhältnis zu seiner Länge relativ dünn ist. Eine Stange kann rund, jedoch auch eckig, insbesondere vieleckig, beispielsweise hexagonal, ausgestaltet sein. Der Begriff umfasst sowohl massive Gegenstände ohne Einkerbungen als auch hohle, eingekerbte oder mit Aussparungen versehene Gegenstände.

Ein "Rohr" wird beispielsweise als länglicher Hohlkörper verstanden. Somit kann ein Rohr als Stange mit einem Hohlraum entlang der Längsachse verstanden werden. Das Rohr kann insbesondere an seinen Enden eine Öffnung des Hohlraums aufweisen oder zwei Öffnungen an jeweils einem Ende.

Ein "Rohrinnendurchmesser" ist insbesondere der Durchmesser des Hohlraums quer zur Längsachse des Rohrs. Falls das Rohr keine kreisförmige Innenform aufweist, wird unter dem Rohrinnendurchmesser der Durchmesser eines größtmöglichen Kreises verstanden, wobei der Kreis lediglich den Platz des Hohlraums einnimmt und quer zur Längsachse des Rohrs steht.

Ein "Stangendurchmesser" ist insbesondere der Durchmesser der Stange quer zur Längsachse der Stange. Falls die Stange keine kreisförmige Außenform aufweist, wird unter dem Stangendurchmesser der Durchmesser eines kleinstmöglichen Kreises verstanden, wobei der Kreis lediglich die Stange vollkommen umfasst und quer zur Längsachse der Stange steht.

Die "Haltevorrichtung" kontaktiert beispielsweise das Rohr an der Außenseite und hält es in einer definierten Position, wobei insbesondere das Rohr durch die Haltevorrichtung weiterhin begradigt wird.

Die "Spannvorrichtung" kann zum Fixieren der Stange in einer bestimmten Position dienen sowie dazu, eine Zugkraft auf die Stange auszuüben.

Der "Zwischenraum" ist beispielsweise der Hohlraum des Rohrs abzüglich des in das Rohr eingeführten Volumens der Stange.

Die "erhärtende Flüssigkeit" ist beispielsweise eine Substanz, welche bei Raumtemperatur oder bei erhöhten Temperaturen fließfähig ist und so in den Zwischenraum eingeführt oder gegossen werden kann und den Zwischenraum somit im Wesentlichen lückenlos ausfüllen kann sowie anschließend erstarrt ("erhärtet"). Somit handelt es sich bei der erhärtenden Flüssigkeit insbesondere um einen Thermoplasten, beispielsweise Polyamid, oder eine monomere Mischung für einen Duroplasten, beispielsweise ein Epoxid-Harz mit Härter.

Unter einer "Krümmung" kann die lokale Abweichung einer Kurve von einer Geraden verstanden werden. Wenn das Rohr oder die Stange "gekrümmt" sind, weisen sie an einer Stelle eine Kurvenform auf. Ein Maß für diese Krümmung ist beispielsweise der Radius der Kurve, welcher der Kurvenform zu Grunde liegt. Eine geringere Krümmung kann mathematisch also durch eine Vergrößerung des Radius beschrieben werden. Eine geringere Krümmung kann auch als "erhöhte Gradheit" oder "begradigt" bezeichnet werden.

Ein Kern der Erfindung besteht insbesondere darin, dass eine zweipolig elektrisch leitende Zugstange gefertigt wird, in der ein Kurzschluss zwischen dem als einem ersten Pol ausgestalteten Rohr und der als zweiten Pol ausgestalteten Stange vermieden wird. Ein Kurzschluss wird dabei vermieden, indem eine Krümmung der Stange ausgeglichen wird.

Eine geringere Krümmung der Stange wird erreicht, indem auf die Stange eine Zugkraft aufgebracht wird und diese somit quasi gespannt wird.

Sobald die begradigte Stange in dem begradigten Rohr angeordnet ist, kann wenigstens eine Seite des Rohrs beispielsweise mittels einer Scheibe abgedichtet werden und der Zwischenraum zwischen Rohr und Stange mittels der erhärtenden Flüssigkeit ausgefüllt werden. Nach dem Aushärten der erhärtenden Flüssigkeit können die Bauteile wieder entlastet werden, wobei die erhärtende Flüssigkeit, welche jetzt als erhärtete Substanz vorliegt, für eine Trennung von Stange und Rohr sorgt. In einer Ausführungsform wird somit vor dem Einführen der erhärtenden Flüssigkeit eine Öffnung des Rohrs mittels eines Dichtelements verschlossen.

Auf das einseitige Abdichten kann hingegen auch verzichtet werden, falls beispielsweise ein rasch aufschäumender und aushärtender Kunststoff verwendet wird oder eine rasch erhärtende Substanz, welche nicht aus dem unteren Ende herauslaufen kann.

Somit liegt nach Abschluss des vorliegenden Verfahrens eine elektrisch zweipolig leitende Zugstange vor, welche wenigstens eine Stange, ein Rohr und eine erhärtete Substanz aufweist.

Die erhärtende Flüssigkeit kann auch eine aufschäumende Substanz sein, wie beispielsweise expandiertes Polystyrol, wodurch zahlreiche abgeschlossene oder miteinander verbundene Hohlräume in der erhärtenden Substanz gebildet werden.

In einer weiteren Ausführungsform weist die Haltevorrichtung eine Begradigungsvorrichtung mit einer geringeren Krümmung als das Rohr auf, sodass eine Krümmung des Rohrs reduziert wird.

Somit wird das Rohr in die Haltevorrichtung mit Begradigungsvorrichtung eingespannt, sodass insbesondere eine Krümmung des Rohrs durch die geringere Krümmung der Begradigungsvorrichtung reduziert wird. Bei der Begradigungsvorrichtung handelt es sich insbesondere um eine Aussparung, welche wenigstens in Teilen in etwa die Außenmaße des Rohrs aufweist, jedoch eine geringere Krümmung als das Rohr aufweist.

Es wird eine Zugstange mit geringerer Krümmung erhalten, da das Rohr in einer geringer gekrümmten Position mit der erhärtenden Flüssigkeit gefüllt und somit in dieser Position fixiert wird. Eine Zugstange geringerer Krümmung besitzt somit eine erhöhte Qualität und bessere Eignung für weitere Fertigungsschritte.

Das Ausgleichen der Krümmung erfolgt dabei im Falle des Rohrs, indem dieses in eine Form eingespannt oder eingeschoben wird, wobei die Form ein geringeres Maß an Krümmung als das zu begradigende Rohr aufweist. Somit kann die Krümmung des Rohrs und/oder die Krümmung der Stange reduziert werden.

In einer weiteren Ausführungsform sind die Stange und das Rohr aus Metall, insbesondere Edelstahl, gefertigt.

Eine Fertigung aus Metall erlaubt hierbei eine hohe mechanische Stabilität sowie Widerstandsfähigkeit gegenüber aggressiven Medien, wie beispielsweise heißem Wasserdampf während des Sterilisierens. Eine Fertigung aus Metall, Stahl, insb. Edelstahl, ermöglicht weiterhin eine hohe Leitfähigkeit des hieraus gefertigten Gegenstandes.

Um ein geringes Volumen zu erreichen, sind der Rohrinnendurchmesser und der Stangendurchmesser derart ausgewählt, dass ein Abschnitt des Zwischenraums zwischen der Stange und dem Rohr kleiner als ein Durchmesser der Stange ist.

Da Stange und Rohr bei einer Fertigung aus Metall überproportional zur Stabilität der Zugstange beitragen, wird hierdurch die Stabilität oder die Leitfähigkeit deutlich verbessert. Der Zwischenraum wird dabei bevorzugt lediglich in einem solchen Ausmaß verringert, dass die Isolationsfähigkeit der erhärtenden Substanz in der gewählten Schichtdicke ausreichend ist.

In einer weiteren Ausführungsform ist die Zugkraft längs der Stange derart eingestellt, dass die Stange lediglich im elastischen Bereich gedehnt wird.

Unter "elastischer Dehnung" wird der linear-elastische Bereich eines Spannung-Dehnungs-Diagramms verstanden, in welchem die Dehnung der Spannung proportional ist und somit das Hook'sche Gesetz gilt.

Somit geht die Stange nach dem Anlegen der Zugkraft wieder in ihre ursprüngliche Form über, sodass sich der Durchmesser der Stange durch das vorliegende Verfahren nicht ändert. Somit werden andernfalls notwendige, anspruchsvolle Berechnungen zur Kompensation der Längenänderung vermieden. Weiterhin wird das Material weniger belastet.

Um ein geringes Gewicht der Zugstange zu erreichen, ist die erhärtende Flüssigkeit ein Kunststoff über seinem Schmelzpunkt oder ein Monomer eines vernetzenden Kunststoffs.

Unter einem "Kunststoff über seinem Schmelzpunkt" wird ein thermoplastischer Kunststoff verstanden, welcher sich in einem flüssigen oder zumindest formbaren Zustand befindet und durch Abkühlen in einen festen Zustand überführt werden kann. Unter einem "Monomer eines vernetzenden Kunststoffs" wird eine Substanz verstanden, welche nach einer chemischen Vernetzung einen Duroplasten bildet. Dabei kann es sich insbesondere um ein Monomer oder ein Oligomer (Präpolymer) handeln.

Kunststoffe zeichnen sich durch eine geringe Dichte aus, wodurch eine Zugstange mit verhältnismäßig geringem Gewicht realisiert werden kann. Weiterhin besitzen sie eine geringe elektrische Leitfähigkeit, wodurch sie als elektrischer Isolator verwendet werden können.

In einer weiteren Ausführungsform ist die erhärtende Flüssigkeit ein Epoxid-Harz. Epoxid-Harze zeichnen sich durch ausgezeichnete mechanische Eigenschaften sowie eine gute Temperatur- und Chemikalienbeständigkeit aus, wodurch die entsprechenden Bereiche der Zugstange aus Epoxid-Harz diese Eigenschaften aufweisen.

Um eine ausreichende Stabilität für die Verwendung in einem Medizininstrument, insbesondere Zangensystem, speziell HF-Zangensystem, oder auch einem Endoskop oder Exoskop zu erhalten, weist das Rohr einen Rohrinnendurchmesser von 1 mm bis 70 mm auf. Es kann somit beispielsweise eine Stange mit einem Stangendurchmesser von 0.8 mm in das Rohr eingeführt werden.

Somit wird sowohl eine ausreichend geringe Größe realisierbar, mittels welcher die Zugstange in Körperöffnungen eines menschlichen Körpers eingeführt werden kann, als auch eine ausreichende Stabilität der Zugstange sowie eine ausreichende elektrische Leitfähigkeit der Stange gewährleistet.

In einer weiteren Ausführungsform ist wenigstens ein Ende des Rohrs mittels einer Dichtung aus einem nichthaftenden Material, insbesondere Teflon, versiegelt. Neben Teflon sind auch andere fluorierte oder chlorierte Kunststoffe prinzipiell möglich, ebenso wie nicht-haftende Keramiken oder Polysulfone.

Somit wird ein Ausfließen der noch nicht erhärteten erhärtenden Flüssigkeit realisiert, sowie weiterhin eine glatte Unterkante der erhärtenden Substanz sichergestellt. Weiterhin findet keine Haftung statt, sodass ein Entfernen der fertigen Zugstange aus der Fertigungsvorrichtung wenig Kraft erfordert und keine Schäden verursacht.

In einem weiteren Aspekt wird die Aufgabe gelöst durch eine elektrische leitende Zugstange, welche mittels eines oben beschriebenen Verfahrens gefertigt wurde. Somit ergeben sich die oben beschrieben Vorteile, wobei weiterhin die Nachteile des Standes der Technik vermieden werden, insbesondere wird eine hohe Fertigungsqualität ermöglicht und Zwischenräume in der Zugstange werden vermieden.

In einem weiteren Aspekt wird die Aufgabe gelöst durch ein medizinisches Instrument insbesondere Zangensystem, speziell HF-Zangensystem, oder auch Endoskop oder Exoskop, welches eine zuvor beschriebene Zugstange aufweist. Somit werden auch im Falle des medizinischen Instruments die oben beschriebene Vorteile realisiert.

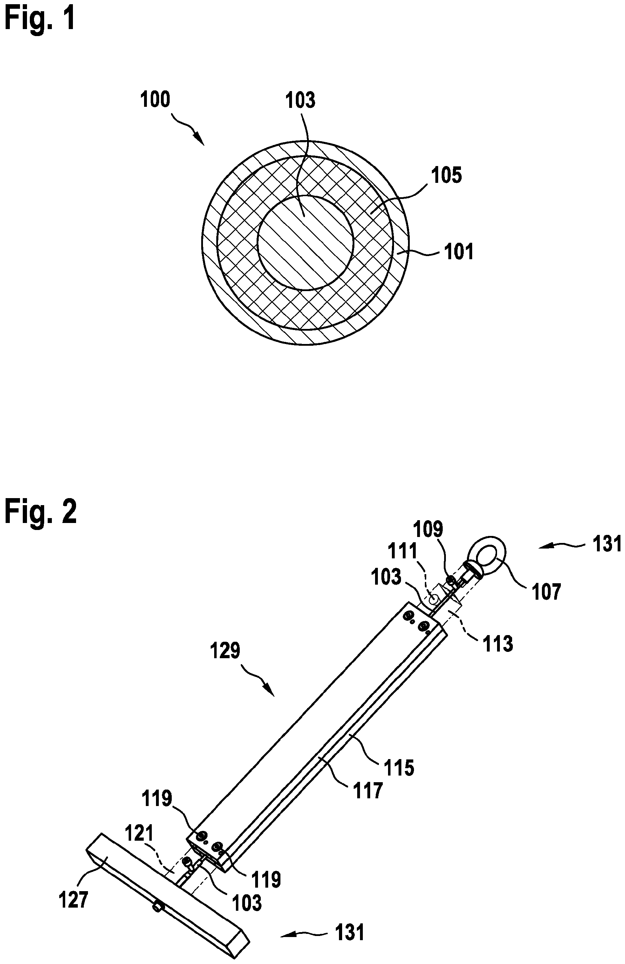

Im Weiteren wird die Erfindung anhand von Ausführungsbeispielen erläutert. Es zeigen
- Figur 1: eine stark schematische Schnitt-Ansicht einer Zugstange mit einem Aluminiumrohr und einer Aluminiumstange, welche mittels Epoxid-Harz verbunden sind,
- Figur 2: eine schematische perspektivische Ansicht einer Rohrausrichtform und eines Stangenspanners mit eingelegtem Aluminiumrohr und eingelegter Aluminiumstange zur Herstellung der Zugstange und

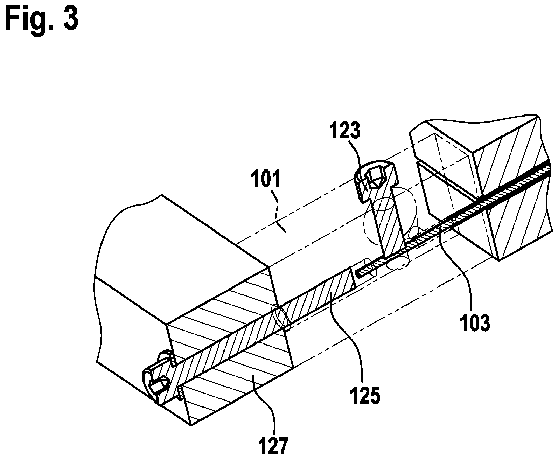

- Figur 3: eine schematische perspektivische Detailansicht von der Rohrausrichtform und dem Stangenspanner mit abgebildetem Aluminiumrohr und abgebildeter Aluminiumstange.

Zum Fertigen einer Zugstange 100, welche zwei elektrische Pole aufweist, wird zunächst die Krümmung eines kreisrunden Aluminiumrohrs 101 reduziert. Dabei wird eine Rohrausrichtform 129 mit einem Innenprisma verwendet, welche eine obere Hälfte 117 und eine untere Hälfte 115 aufweist. Zum Reduzieren der Krümmung wird das Aluminiumrohr 101 in das Innenprisma der unteren Hälfte 115 der Rohrausrichtform 129, welche das Innenprisma aufweist, eingelegt. Anschließend wird die obere Hälfte 117 der als Rohrausrichtform 129 auf die untere Hälfte 115 derart gelegt, dass das Aluminiumrohr 101 vollständig durch die untere Hälfte 115 und die obere Hälfte 117 der Rohrausrichtform 129 umgeben ist.

Das Aluminiumrohr 101 ist etwas länger als die Rohrausrichtform 129 mit Innenprisma, sodass das Aluminiumrohr 101 an zwei Seiten aus dem Innenprisma herausragt. Das Innenprisma der Rohrausrichtform 129 ist mit hoher Präzision geradlinig gefertigt, sodass das Aluminiumrohr 101 geradlinig ausgerichtet wird.

Der Rohrausrichtform 129 mit eingelegtem Aluminiumrohr 101 wird anschließend an einem Gerüst fixiert. In das in die Rohrausrichtform 129 eingespannte Aluminiumrohr 101 wird die Aluminiumstange 103 eingeführt. Die Längsachse des Aluminiumrohrs 101 und die Längsachse der Aluminiumstange 103 liegen jetzt in etwa übereinander.

Die eingeführte Aluminiumstange 103 wird dann mit einem Stangenspanner 131 verbunden. Der Stangenspanner 131 weist eine Augenschraube 107 zum Aufbringen der Zugkraft, eine obere Stangenschraube 109 und eine obere Teflon-Dichtung 113 auf.

Auf die Aluminiumstange 103 wird eine untere Teflon-Dichtung 121 mit einem Gegengewicht 127, beispielsweise aus Metall, insb. Eisen bzw. Stahl, aufgeschoben. Die Aluminiumstange 103 wird dann in der unteren Teflon-Dichtung 121 mittels einer unteren Stangenschraube 123 fest in der unteren Teflon-Dichtung 121 verankert. Die untere Teflon-Dichtung 121 wird an dem Gegengewicht 127 mittels einer Dichtungsschraube 125 festgehalten. Somit ist das Aluminiumrohr 101 an einem unteren Ende mittels der Dichtungsschraube 125 und der unteren Stangenschraube 123 fest mit der unteren Teflon-Dichtung 121 und dem Gegengewicht 127 verbunden.

Auf die Aluminiumstange 103 wird an einem oberen Ende die obere Teflon-Dichtung 113 aufgesetzt, welche mit einer Augenschraube 107 mit dem Aluminiumrohr 101 spannend verbunden ist. Die Aluminiumstange 103 wird in der oberen Teflon-Dichtung 113 mittels der oberen Stangenschraube 109 befestigt. Die Aluminiumstange 103 ist somit an zwei Seiten verankert.

Ein Rohrinnendurchmesser des Aluminiumrohrs 101 ist größer als ein Aluminiumstangenaußendurchmesser der Aluminiumstange 103. Da der Aluminiumstangenaußendurchmesser kleiner als der Aluminiumrohrinnendurchmesser ist und das Aluminiumrohr 101 mittels des Stangenspanners 131 mittig zum Aluminiumrohr 101 ausgerichtet wurde, ist ein etwa gleichmäßiger Zwischenraum um die Aluminiumstange 103 herum in dem Aluminiumrohr 101 vorhanden. Das Aluminiumrohr 101 ist jedoch nicht vollständig gerade gefertigt, sodass es am oberen Ende mit dem Aluminiumrohr 101 in Kontakt tritt.

Mittels einer Seilwinde wird auf die Augenschraube 107 eine definierte Federkraft aufgebracht, welche somit auf die Aluminiumstange 103 als Zugkraft übertragen wird. Durch die auf die Aluminiumstange 103 anlegende Zugkraft richtet diese sich vollständig gerade aus und weist damit keinen Kontakt mehr zum Aluminiumrohr 101 auf.

In eine Einlassöffnung 111 für Epoxid-Harz 105 in der oberen Teflon-Dichtung 113 wird ein Epoxid-Harz eingefüllt, welches in den Zwischenraum zwischen der Aluminiumstange 103 und dem Aluminiumrohr 101 fließt.

Das Epoxid-Harz härtet bei Raumtemperatur innerhalb von 24 Stunden aus, sodass die Aluminiumstange 103 in der zentrierten Position im Aluminiumrohr 101 fixiert wird. Nun wird die fertig hergestellte Zugstange 100 zunächst aus dem Stangenspanner 131 und dann aus der Rohrausrichtform 129 mit Innenprisma herausgenommen.

Die Zugstange 100 wird in einem Zangensystem, insbesondere HF-Zangensystem, speziell für den Betrieb im HF Cut & Koagulation Modus, zum Betreiben eines distal angeordneten Greifers verwendet, wobei der Greifer elektrisch betrieben wird und elektrische Energie über die beiden Pole der Zugstange 100 erhält.

### Bezugszeichenliste

- 100: Zugstange
- 101: Aluminiumrohr
- 103: Aluminiumstange
- 105: Epoxid-Harz
- 107: Augenschraube zum Aufbringen der Zugkraft
- 109: obere Stangenschraube
- 111: Einlassöffnung für Epoxid-Harz
- 113: obere Teflon-Dichtung
- 115: untere Hälfte einer Form mit Innenprisma
- 117: obere Hälfte einer Form mit Innenprisma
- 119: Halteschraube der Form mit Innenprisma
- 121: untere Teflon-Dichtung
- 123: untere Stangenschraube
- 125: Dichtungsschraube
- 127: Gegengewicht
- 129: Rohrausrichtform mit Innenprisma
- 131: Stangenspanner

## Patentansprüche

1. Verfahren zum Herstellen einer zweipolig elektrisch leitenden Zugstange mit einer elektrisch leitenden Stange in einem elektrisch leitenden Rohr, wobei ein Rohrinnendurchmesser größer als ein Stangendurchmesser ist, mit den folgenden Schritten:
- Einführen des Rohrs in eine Haltevorrichtung, wobei die Haltevorrichtung derart ausgestaltet ist, dass die Haltevorrichtung das Rohr an einer Rohraußenseite kontaktiert,
- Befestigen der Stange in einer Spannvorrichtung, wobei die Stange mittels der Spannvorrichtung beim Befestigen derart in Bezug zur Haltevorrichtung ausgerichtet wird, dass die befestigte Stange mittels der Spannvorrichtung mittig in das Rohr einführbar bzw. ausrichtbar ist,
- mittiges Einführen bzw. Ausrichten der befestigten Stange in das Rohr, wobei eine Rohrinnenseite und eine Stangenaußenseite der eingeführten Stange vollständig durch einen Zwischenraum getrennt sind, sodass die Stange und das Rohr frei von Kontakt sind, wobei die Stange entweder vor oder nach dem Einführen mittels der Spannvorrichtung gespannt wird, sodass eine Zugkraft längs der Stange wirkt, und
- Ausfüllen des Zwischenraums mit einer erhärtenden Flüssigkeit bzw. Granulat, insb. Kunststoffgranulat, wobei die erhärtende Flüssigkeit wenigstens im erhärteten Zustand isolierend wirkt, sodass die Stange mittig in dem Rohr fixiert und die zweipolig elektrisch leitende Zugstange erhalten wird.

2. Verfahren nach Anspruch 1, **dadurch gekennzeichnet, dass** die Haltevorrichtung eine Begradigungsvorrichtung mit einer geringeren Krümmung als das Rohr aufweist, sodass eine Krümmung des Rohrs reduziert wird.

3. Verfahren nach einem der vorherigen Ansprüche, **dadurch gekennzeichnet, dass** die Stange und das Rohr aus Metall, insbesondere Stahl bzw. Edelstahl, gefertigt sind.

4. Verfahren nach einem der vorherigen Ansprüche, **dadurch gekennzeichnet, dass** der Rohrinnendurchmesser und der Stangendurchmesser derart gewählt sind, dass ein Abschnitt des Zwischenraums zwischen der Stange und dem Rohr kleiner als ein Durchmesser der Stange ist.

5. Verfahren nach einem der vorherigen Ansprüche, **dadurch gekennzeichnet, dass** die Zugkraft längs der Stange derart eingestellt ist, dass die Stange lediglich im elastischen Bereich gedehnt wird.

6. Verfahren nach einem der vorherigen Ansprüche, **dadurch gekennzeichnet, dass** die erhärtende Flüssigkeit bzw. das Granulat ein Kunststoff über seinem Schmelzpunkt oder ein Monomer eines vernetzenden Kunststoffs ist.

7. Verfahren nach Anspruch 5, **dadurch gekennzeichnet, dass** die erhärtende Flüssigkeit ein Epoxid-Harz ist.

8. Verfahren nach einem der vorherigen Ansprüche, **dadurch gekennzeichnet, dass** das Rohr einen Rohrinnendurchmesser von 1 mm bis 70 mm aufweist.

9. Verfahren nach einem der vorherigen Ansprüche, **dadurch gekennzeichnet, dass** wenigstens ein Ende des Rohrs mittels einer Dichtung aus einem nichthaftenden Material, insbesondere Teflon, versiegelt ist.

10. Elektrisch leitenden Zugstange, welche mittels eines Verfahrens nach einem der vorherigen Ansprüche gefertigt wurde.

11. Medizinisches Instrument, insbesondere Zangensystem, speziell HF-Zangensystem, oder auch Endoskop oder Exoskop, welches eine Zugstange nach Anspruch 10 aufweist.
